# EUROPEAN PATENT APPLICATION

(11) **EP 3 988 560 A1**
(43) Date of publication of application: **27.04.2022**
(21) Application number: 20825935.8
(22) Date of filing: 22.06.2020
(51) Int. Cl.: C07H 19/167, A61K 31/7076, A61P 9/10

(54) **SMALL-MOLECULE COMPOUND HAVING A2A ADENOSINE RECEPTOR ANTAGONISM**

(30) Priority: 21.06.2019 CN 201910542437
(71) Applicant: Academy Of Military Medical Sciences, Haidian District Beijing 100850 (CN)
(72) Inventor: ZHONG, Wu, Beijing 100850 (CN); ZHANG, Min, Beijing 100850 (CN); ZHOU, Xinbo, Beijing 100850 (CN); FAN, Shiyong, Beijing 100850 (CN); LI, Song, Beijing 100850 (CN)
(74) Representative: Böhm, Brigitte
(86) International application number: PCT/CN2020/097393
(87) International publication number: WO 2020/253867

(57) **Abstract**

The present application provides a small-molecule compound, represented by general Formula (I), having A_{2A} adenosine receptor antagonistic activity and a pharmaceutical composition containing same. The compound and composition can be used as A_{2A} adenosine receptor antagonistic agents.

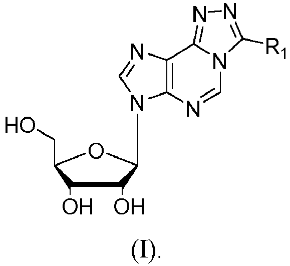

## Description

The present application is based on and claims the benefit of priority from Chinese application No. 201910542437.0, filed on June 21, 2019, the disclosures of which are incorporated herein by reference in its entirety.

### Technical Field

The present application belongs to the field of medicine technology, specifically relates to a small molecule compound having A_{2A} adenosine receptor antagonism and a pharmaceutical composition containing the same. The compound and composition can be used as a medicament.

### Background Art

Adenosine is a well-known intermediate of purine synthesis and energy metabolism, and one of the most important neuromodulators in the human body, the target receptor which act on is adenosine receptor (AR). Adenosine receptor is a G protein-coupled receptor (GPCR), and is divided into four categories in pharmacology: A₁, A_{2A}, A_{2B} and A₃ (FREDHOLM B B, et al., Pharmacol Rev, 2011, 63: 1-34; FREDHOLM BB, et al., Exp Cell Res, 2010, 316: 1284-8). Among them, A_{1A}R and A_{3A}R couple to Gi protein and inhibit adenylate cyclase, while A_{2A}AR and A_{2B}AR couple to Gs protein and stimulate adenylate cyclase. The interaction between A_{2A}AR and G protein makes GTP bind to G protein α subunit, and at the same time β/γ subunits dissociate, thereby promoting the production of cAMP. A_{2A}AR plays a key role in a variety of pathophysiological and physiological processes. Physiologically, A_{2A}AR plays an important role in regulating vasodilation and supports the synthesis of new blood vessels. A_{2A}AR regulates myocardial blood flow by dilation of coronary arteries and increases myocardial blood flow, but it may cause hypotension. The key role of A_{2A}AR under various pathological conditions, especially in the treatment of immunosuppressive diseases, inflammatory tissue damage, and neurodegenerative diseases, such as Alzheimer's disease, Huntington's disease and Parkinson's disease (PD), etc., has been extensively studied (DE LERA RUIZ M et al., J Med Chem, 2014, 57: 3623-50; CHEN JF et al., Nat Rev Drug Discov, 2013, 12: 265-86; MULLER C E et al., Biochim Biophys Acta, 2011, 1808: 1290-308; YUAN G et al., Curr Med Chem, 2014, 21: 3918-35). Existing studies have shown that A_{2A}AR is of great significance in regulating inflammation, immune response and neuroprotection (CHEN J F et al., Nat Rev Drug Discov, 2013, 12: 265-86; LAPPAS C M et al., Expert Opin Investig Drugs, 2005, 14: 797-806; VARANI K et al., Autoimmun Rev, 2010, 10: 61-4). A_{2A}AR is expressed in a variety of organs, tissues and cells. In the central nervous system, A_{2A}AR is highly expressed in the dopamine functional areas, the GABAergic medium spiny neurons of dorsal striatum, the neurons in the core and shell of nucleus accumbens, and the olfactory tubercle of the brain. In the peripheral system, A_{2A}AR is highly expressed in heart, liver, lung, spleen and thymus, etc. In addition, A_{2A}AR is also highly expressed in intestinal mucosa, platelets, various immune cells, and vascular endothelial cells.

In the past few years, A_{2A}AR antagonists have been considered as neuroprotective pharmacological tools (Cristalli G et al., ChemMedChem 2007, 2: 260-81; Cacciari B et al., Curr Top Med Chem 2003, 3: 403-11; Moro S Et al. Med Res Rev 2006, 26: 131-59). Due to the wide distribution of A_{2A}AR in the human body, A_{2A}AR antagonists are recommended for the treatment of various pathological diseases (patent application: CN201180022061.2). A_{2A}AR is located in striatum and is co-expressed with dopamine D2 receptor, and they have been proven to be attractive targets for the treatment of several central nervous system diseases, such as movement disorders and Parkinson's disease (Schwarzschild MA et al., Trends Neurosci 2006, 29: 647-54; Jacobson KA et al., Nat Rev Drug Discov 2006, 5: 247-64; Impagnatiello F et al., Emerg Ther Targets 2000, 4: 635-63; Fuxe K et al., Mov Disord 2007, 22: 1990-2017; Schapira AH et al., Nat Rev Drug Discov 2006, 5: 845-54; Xu K et al., Pharmacol Ther 2005, 105: 267-310). In addition, A_{2A}AR antagonists seem to protect cells from ischemia-induced death, and may also act as cognitive enhancers, drugs with neuroprotective effect and anti-allergic action, analgesics and positive inotropic drugs, and even are used for the treatment of alcoholism (Melani A et al., Brain Res 2006, 1073-1074: 470-80; Monopoli A et al., Neuroreport 1998, 9: 3955-9; Richardson PJ et al., Trends Pharmacol Sci 1997, 18: 338-44; Pedata F et al., Neurol Res 2005, 27: 169-74; Ledent C et al., Nature 1997, 388: 674-8; Thorsell A et al., Alcohol Clin Exp Res 2007, 31: 1302-7; Brambilla R et al., Glia 2003, 43: 190-4; Takahashi RN et al., Front Biosci 2008, 13: 2614-32). These pharmacological results strongly support the development of A_{2A}AR antagonists (Yang M et al., Naunyn Schmiedeberg's Arch Pharmacol 2007, 375: 133-44; Yuzlenko O et al., Curr Med Chem 2006, 13: 3609-25).

Although the above-mentioned A_{2A}AR antagonists have been increasingly developed, no relevant new drugs have been approved for marketing through clinical trials. Therefore, there is still a need in the art for structurally novel and effective A_{2A} receptor antagonists with optionally one or more physiological and/or physicochemical advantages, and it is important to continuously synthesize and test new A_{2A} receptor antagonists in order to develop new and improved therapeutic agents.

### Contents of the present invention

The purpose of the present application is to find and develop a new type of small molecule antagonist that act on A_{2A} adenosine receptor, which can antagonize A_{2A} adenosine receptor, so as to achieve the purpose of preventing or treating a human pathological condition or symptom, and the prevention or treatment of human pathological condition or symptom involves the activity of A_{2A} adenosine receptor and the antagonism of this activity is required. In the present application, it has been found through research that the compound with the following general Formula I can act on A_{2A} adenosine receptor and is an A_{2A} adenosine receptor antagonist, and therefore can be used for the above purposes. The present application was completed based on the above findings.

Therefore, the first aspect of the present application provides a compound represented by Formula (I), a stereoisomer, a pharmaceutically acceptable salt, a pharmaceutically acceptable hydrate or solvate, or a pharmaceutically acceptable ester thereof, 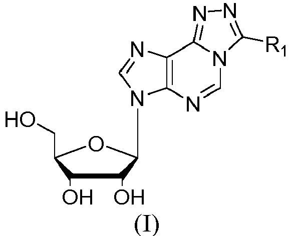 wherein:
R₁ is selected from the group consisting of aryl, cycloalkyl, heteroaryl, heterocycloalkyl, aryl substituted by one or more substituents R¹⁰, cycloalkyl substituted by one or more substituents R¹⁰, heteroaryl substituted by one or more substituents R¹⁰, and heterocycloalkyl substituted by one or more substituents R¹⁰, wherein R¹⁰ is selected from the group consisting of halogen, C₁₋₆ alkoxy, C₁₋₆ alkyl, hydroxyl, amino, cyano, nitro, halogenated C₁₋₆ alkyl, - NHC(O)R¹¹, benzyloxy, halogenated phenylmethoxy, aryl and heteroaryl, R¹¹ is C₁₋₆ alkyl (e.g., methyl, ethyl, n-propyl, isopropyl, n-butyl, isobutyl, tert-butyl, n-pentyl, tert-pentyl, neopentyl, hexyl).

According to some embodiments of the present application, R¹¹ in Formula (I) is C₁₋₄ alkyl.

According to some embodiments of the present application, R¹¹ in Formula (I) is methyl, ethyl, n-propyl, isopropyl, n-butyl, isobutyl, tert-butyl, n-pentyl, tert-pentyl, neopentyl or hexyl.

According to some embodiments of the present application, R₁ in Formula (I) is selected from the group consisting of phenyl, cyclopentyl, cyclohexyl, pyridyl, thiazolyl, pyrrolyl, imidazolyl, furanyl, phenyl substituted by one or more substituents R¹⁰, cyclopentyl substituted by one or more substituents R¹⁰, cyclohexyl substituted by one or more substituents R¹⁰, pyridyl substituted by one or more substituents R¹⁰, thiazolyl substituted by one or more substituents R¹⁰, pyrrolyl substituted by one or more substituents R¹⁰, imidazolyl substituted by one or more substituents R¹⁰, and furanyl substituted by one or more substituents R¹⁰.

According to some embodiments of the present application, R₁ in Formula (I) is selected from the group consisting of phenyl, cyclopentyl, cyclohexyl, pyridin-2-yl, thiazol-5-yl, phenyl substituted by one or more substituents R¹⁰, cyclopentyl substituted by one or more substituents R¹⁰, cyclohexyl substituted by one or more substituents R¹⁰, pyridin-2-yl substituted by one or more substituents R¹⁰, thiazol-5-yl substituted by one or more substituents R¹⁰.

According to some embodiments of the present application, R¹⁰ in Formula (I) is selected from the group consisting of halogen, C₁₋₄ alkoxy, C₁₋₄ alkyl, hydroxyl, amino, cyano, nitro, halogenated C₁₋₄ alkyl, -NHC(O)R¹¹, benzyloxy, halogenated phenylmethoxy, aryl, and heteroaryl, R¹¹ is C₁₋₄ alkyl, or
according to some embodiments of the present application, R¹⁰ in Formula (I) is selected from the group consisting of fluorine, chlorine, bromine, iodine, methyl, ethyl, n-propyl, isopropyl, n-butyl, isobutyl, tert-butyl, n-pentyl, tert-pentyl, neopentyl, hexyl, trifluoromethyl, difluoromethyl, fluoromethyl, methoxy, ethoxy, n-propoxy, isopropoxy, n-butoxy, tert-butoxy, sec-butoxy, n-pentyloxy, n-hexyloxy, hydroxyl, amino, cyano, nitro, acetamido, formylamino, propionamido, benzyloxy, fluorophenylmethoxy, phenyl, pyridyl, pyrrolidinyl, cyclopentyl, cyclohexyl, morpholinyl, imidazolyl, and thiazolyl.

According to some embodiments of the present application, R¹⁰ in Formula (I) is selected from the group consisting of fluorine, chlorine, bromine, methyl, ethyl, n-propyl, isopropyl, n-butyl, isobutyl, tert-butyl, trifluoromethyl, methoxy, ethoxy, n-propoxy, isopropoxy, n-butoxy, tert-butoxy, sec-butoxy, hydroxyl, amino, cyano, nitro, acetamido, formamido, propionamido, benzyloxy, 4-fluorophenylmethoxy, phenyl, pyridin-2-yl, 5-bromopyridin-2-yl, cyclopentyl, and cyclohexyl.

According to some embodiments of the present application, R₁ in Formula (I) is 4-acetamidophenyl, cyclopentyl, 3,4-dibenzyloxyphenyl, 4-(4-fluorobenzyloxy)phenyl, 3-benzyloxyphenyl, 2,4-di(trifluoromethyl)phenyl, 4-(pyridin-2-yl)phenyl, 4-phenylphenyl, 4-propoxyphenyl, 4-trifluoromethylphenyl, 5-bromopyridin-2-yl, thiazol-5-yl or cyclohexyl.

According to some embodiments of the present application, R₁ in Formula (I) is phenyl or phenyl substituted by one or more substituents R¹⁰, and R¹⁰ is selected from the group consisting of halogen, -NHC(O)R¹¹, C₁₋₆ alkoxy, C₁₋₆ alkyl, hydroxyl, amino, cyano, nitro, halogenated C₁₋₆ alkyl, benzyloxy, halogenated phenylmethoxy, aryl, heteroaryl, R¹¹ is C₁₋₆ alkyl (e.g. methyl, ethyl, n-propyl, isopropyl, n-butyl, isobutyl, tert-butyl, n-pentyl, tert-pentyl, neopentyl, hexyl).

According to some embodiments of the present application, R₁ in Formula (I) is selected from the group consisting of cyclopentyl, thiazolyl, and phenyl para- or meta-substituted by substituent R¹⁰, wherein R¹⁰ is selected from the group consisting of -NHC(O)R¹¹, C₁₋₆ alkoxy, benzyloxy and halogenated benzyloxy, R¹¹ is C₁₋₆ alkyl.

According to some embodiments of the present application, R₁ in Formula (I) is selected from the group consisting of cyclopentyl, thiazol-5-yl, and phenyl para- or meta-substituted by substituent R¹⁰, wherein R¹⁰ is selected from the group consisting of -NHC(O) R¹¹, C₁₋₆ alkoxy, benzyloxy and halogenated benzyloxy, R¹¹ is C₁₋₆ alkyl.

According to some embodiments of the present application, R₁ in Formula (I) is selected from the group consisting of cyclopentyl, thiazol-5-yl, and phenyl para- or meat-substituted by substituent R¹⁰, wherein R¹⁰ is selected from the group consisting of -NHC(O) R¹¹, C₁₋₄ alkoxy, benzyloxy and halogenated benzyloxy, R¹¹ is C₁₋₄ alkyl.

According to some embodiments of the present application, R₁ in Formula (I) is cyclopentyl.

According to some embodiments of the present application, R₁ in Formula (I) is thiazol-5-yl.

According to some embodiments of the present application, R₁ in Formula (I) is phenyl para-substituted by substituent R¹⁰, wherein R¹⁰ is selected from the group consisting of - NHC(O)R¹¹, C₁₋₄ alkoxy, benzyloxy and halogenated benzyloxy, R¹¹ is C₁₋₄ alkyl.

According to some embodiments of the present application, R₁ in Formula (I) is phenyl para-substituted by substituent R¹⁰, wherein R¹⁰ is benzyloxy.

According to some embodiments of the present application, R₁ in Formula (I) is phenyl para-substituted by substituent R¹⁰, wherein R¹⁰ is halogenated benzyloxy.

According to some embodiments of the present application, R₁ in Formula (I) is phenyl para-substituted by substituent R¹⁰, wherein R¹⁰ is chlorobenzyloxy.

According to some embodiments of the present application, R₁ in Formula (I) is phenyl para-substituted by substituent R¹⁰, wherein R¹⁰ is 4-chlorobenzyloxy.

According to some embodiments of the present application, R₁ in Formula (I) is phenyl para-substituted by substituent R¹⁰, wherein R¹⁰ is bromobenzyloxy.

According to some embodiments of the present application, R₁ in Formula (I) is phenyl para-substituted by substituent R¹⁰, wherein R¹⁰ is 4-bromobenzyloxy.

According to some embodiments of the present application, R₁ in Formula (I) is phenyl para-substituted by substituent R¹⁰, wherein R¹⁰ is fluorobenzyloxy.

According to some embodiments of the present application, R₁ in Formula (I) is phenyl para-substituted by substituent R¹⁰, wherein R¹⁰ is 4-fluorobenzyloxy.

According to some embodiments of the present application, R₁ in Formula (I) is phenyl para-substituted by substituent R¹⁰, wherein R¹⁰ is benzyloxy.

According to some embodiments of the present application, R₁ in Formula (I) is phenyl para-substituted by substituent R¹⁰, wherein R¹⁰ is C₁₋₆ alkoxy.

According to some embodiments of the present application, R₁ in Formula (I) is phenyl para-substituted by substituent R¹⁰, wherein R¹⁰ is C₁₋₄ alkoxy.

According to some embodiments of the present application, R₁ in Formula (I) is phenyl para-substituted by substituent R¹⁰, wherein R¹⁰ is methoxy, ethoxy, n-propoxy, isopropoxy, n-butoxy, tert-butoxy, sec-butoxy, n-pentyloxy, tert-pentyloxy, neopentyloxy, or hexyloxy.

According to some embodiments of the present application, R₁ in Formula (I) is phenyl para-substituted by substituent R¹⁰, wherein R¹⁰ is methoxy, ethoxy, n-propoxy, n-butoxy, n-pentyloxy, or hexyloxy.

According to some embodiments of the present application, R₁ in Formula (I) is phenyl para-substituted by substituent R¹⁰, wherein R¹⁰ is methoxy, ethoxy, n-propoxy, or n-butoxy.

According to some embodiments of the present application, R₁ in Formula (I) is phenyl para-substituted by substituent R¹⁰, wherein R¹⁰ is methoxy.

According to some embodiments of the present application, R₁ in Formula (I) is phenyl para-substituted by substituent R¹⁰, wherein R¹⁰ is ethoxy.

According to some embodiments of the present application, R₁ in Formula (I) is phenyl para-substituted by substituent R¹⁰, wherein R¹⁰ is n-propoxy.

According to some embodiments of the present application, R₁ in Formula (I) is phenyl para-substituted by substituent R¹⁰, wherein R¹⁰ is n-butoxy.

According to some embodiments of the present application, R₁ in Formula (I) is phenyl meta-substituted by substituent R¹⁰, wherein R¹⁰ is selected from the group consisting of - NHC(O)R¹¹, C₁₋₄ alkoxy, benzyloxy and halogenated benzyloxy, R¹¹ is C₁₋₄ alkyl.

According to some embodiments of the present application, R¹¹ in the substituent - NHC(O)R¹¹ is methyl.

According to some embodiments of the present application, R¹¹ in the substituent - NHC(O)R¹¹ is ethyl.

According to some embodiments of the present application, R¹¹ in the substituent - NHC(O)R¹¹ is n-propyl.

According to some embodiments of the present application, R¹¹ in the substituent - NHC(O)R¹¹ is isopropyl.

According to some embodiments of the present application, R¹¹ in the substituent - NHC(O)R¹¹ is n-butyl.

According to some embodiments of the present application, R¹¹ in the substituent - NHC(O)R¹¹ is isobutyl.

According to some embodiments of the present application, R¹¹ in the substituent - NHC(O)R¹¹ is tert-butyl.

According to some embodiments of the present application, R¹¹ in the substituent - NHC(O)R¹¹ is n-pentyl.

According to some embodiments of the present application, R¹¹ in the substituent - NHC(O)R¹¹ is tert-pentyl.

According to some embodiments of the present application, R¹¹ in the substituent - NHC(O)R¹¹ is neopentyl.

According to some embodiments of the present application, R¹¹ in the substituent - NHC(O)R¹¹ is hexyl.

According to some embodiments of the present application, R₁ in Formula (I) is phenyl meta-substituted by substituent R¹⁰, wherein R¹⁰ is benzyloxy.

According to some embodiments of the present application, R₁ in Formula (I) is 4-acetamidophenyl, cyclopentyl, 4-(4-fluorobenzyloxy)phenyl, 3-benzyloxyphenyl, 4-propoxyphenyl or thiazol-5-yl.

According to some embodiments of the present application, the compound represented by Formula (I) is selected from the group consisting of:
N-{4-{7-[(2R,3R,4S,5R)-3,4-dihydroxy-5-(hydroxymethyl)tetrahydrofuran-2-yl]-7H-[1,2,4]triazolo[3,4-i]purin-3-yl}phenyl}acetamide;
(2R,3R,4S,5R)-2-{3-cyclopentyl-7H-[1,2,4]triazolo[3,4-i]purin-7-yl}-5-(hydroxymethyl)tetrahydrofuran-3,4-diol;
(2R,3R,4S,5R)-2-{3-[3,4-di(benzyloxy)phenyl]-7H-[1,2,4]triazolo[3,4-i]purin-7-yl}-5-(hydroxymethyl)tetrahydrofuran-3,4-diol;
(2R,3R,4S,5R)-2-{3-{4-[(4-fluorobenzyl)oxy]phenyl}-7H-[1,2,4]triazolo[3,4-i]purin-7-yl}-5-(hydroxymethyl)tetrahydrofuran-3,4-diol;
(2R,3R,4S,5R)-2-{3-[3-(benzyloxy)phenyl]-7H-[1,2,4]triazolo[3,4-i]purin-7-yl}-5-(hydroxymethyl)tetrahydrofuran-3,4-diol;
(2R,3R,4S,5R)-2-{3-[2,4-di(trifluoromethyl)phenyl]-7H-[1,2,4]triazolo[3,4-i]purin-7-yl}-5-(hydroxymethyl)tetrahydrofuran-3,4-diol;
(2R,3S,4R,5R)-2-{3-[4-(pyridin-2-yl)phenyl]-7H-[1,2,4]triazolo[3,4-i]purin-7-yl}-5-(hydroxymethyl)tetrahydrofuran-3,4-diol;
(2R,3R,4S,5R)-2-{3-{[1,1'-biphenyl]-4-yl}-7H-[1,2,4]triazolo[3,4-i]purin-7-yl}-5-(hydroxymethyl)tetrahydrofuran-3,4-diol;
(2R,3S,4R,5R)-2-{3-(4-propoxyphenyl)-7H-[1,2,4]triazolo[3,4-i]purin-7-yl}-5-(hydroxymethyl)tetrahydrofuran-3,4-diol;
(2R,3S,4R,5R)-2-{3-[4-(trifluoromethyl)phenyl]-7H-[1,2,4]triazolo[3,4-i]purin-7-yl}-5-(hydroxymethyl)tetrahydrofuran-3,4-diol;
(2R,3R,4S,5R)-2-{3-(5-bromopyridin-2-yl)-7H-[1,2,4]triazolo[3,4-i]purin-7-yl}-5-(hydroxymethyl)tetrahydrofuran-3,4-diol;
(2R,3S,4R,5R)-2-{3-(thiazol-5-yl)-7H-[1,2,4]triazolo[3,4-i]purin-7-yl}-5-(hydroxymethyl)tetrahydrofuran-3,4-diol; and
(2R,3R,4S,5R)-2-{3-cyclohexyl-7H-[1,2,4]triazolo[3,4-i]purin-7-yl }-5-(hydroxymethyl)tetrahydrofuran-3,4-diol.

The second aspect of the present application provides a method for preparing the compound represented by Formula (I), a stereoisomer, a pharmaceutically acceptable salt, a pharmaceutically acceptable hydrate or solvate, or a pharmaceutically acceptable ester thereof as described in the first aspect of the present application, comprising: 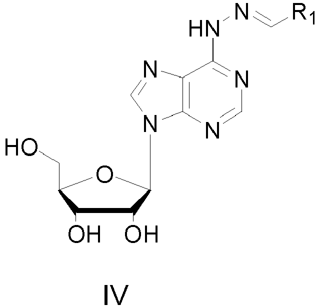 allowing a compound represented by Formula IV to undergo a cyclization reaction to obtain the compound represented by Formula (I), wherein the definition of R₁ is the same as that described in the first aspect of the present application.

According to some embodiments of the present application, the cyclization reaction is carried out in an acetic acid solution and in the presence of NBS.

According to some embodiments of the present application, the cyclization reaction is carried out at room temperature.

According to some embodiments of the present application, the compound represented by Formula IV is obtained by reacting a compound represented by Formula II with a substituted formaldehyde represented by Formula III, wherein the definition of R₁ is the same as that described in the first aspect of the present application.

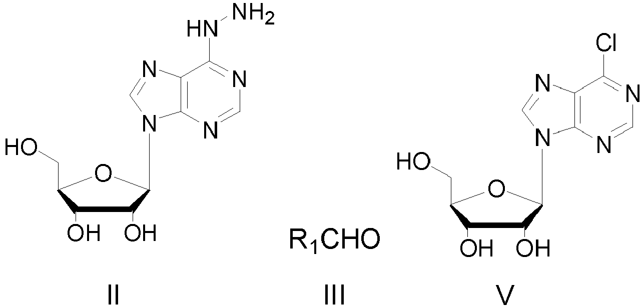

According to some embodiments of the present application, the compound represented by Formula II reacts with the substituted formaldehyde represented by Formula III in a methanol solution under microwaves at 70°C to 90°C, wherein the definition of R₁ is the same as that described in the first aspect of the present application.

According to some embodiments of the present application, the compound represented by Formula II is obtained by the hydrazinolysis reaction of 6-chloroadenosine represented by Formula V with hydrazine hydrate.

According to some embodiments of the present application, the hydrazinolysis reaction is carried out at 60°C to 80°C.

According to some embodiments of the present application, the method as described in the second aspect of the present application has a synthesis reaction process as follows:

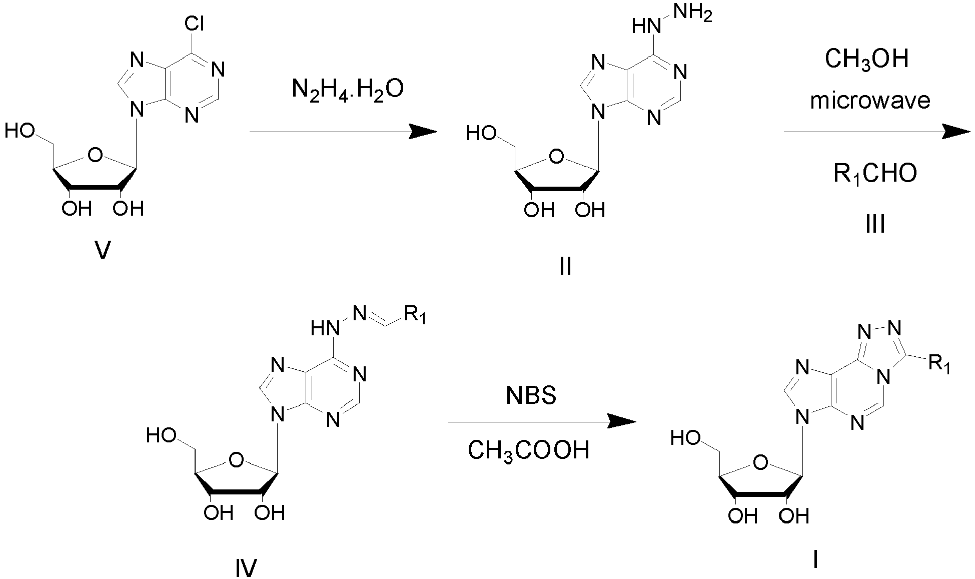

Hydrazinolysis reaction of 6-chloroadenosine (Compound of Formula V) as starting material with hydrazine hydrate is carried out at 60°C to 80°C to produce 6-hydrazinoadenosine (Compound of Formula II); 6-hydrazinoadenosine (Compound of Formula II) reacts with a substituted formaldehyde (Compound of Formula III) in methanol solution under microwaves at 70°C to 90°C to obtain a 6-hydrazinoadenosine compound (Compound of Formula IV); finally, the 6-hydrazinoadenosine compound (Compound of Formula IV) is added to an acetic acid solution, and NBS was added, a cyclization reaction is carried out at room temperature to obtain the compound of Formula (I), wherein the definition of R₁ is the same as that of the compound in the first aspect of the present application.

The third aspect of the present application provides a pharmaceutical composition, which comprises at least one of the compound represented by Formula (I), a stereoisomer, a pharmaceutically acceptable salt, a pharmaceutically acceptable hydrate or solvate, or a pharmaceutically acceptable ester thereof as described in any one of embodiments of the first aspect of the present application, and one or more pharmaceutically acceptable carriers or excipients.

The third aspect of the present application provides use of the compound represented by Formula (I), a stereoisomer, a pharmaceutically acceptable salt, a pharmaceutically acceptable hydrate or solvate, or a pharmaceutically acceptable ester thereof as described in the first aspect of the present application, or the pharmaceutical composition as described in the third aspect of the present application in the manufacture of a medicament as an A_{2A} adenosine receptor antagonist, or
in the manufacture of a medicament for preventing or treating a human pathological condition or symptom, wherein the human pathological condition or symptom is improved by antagonizing A_{2A} adenosine receptor.

The fourth aspect of the present application provides a method for preventing and/or treating a human pathological condition or symptom, comprising administering to a subject in need thereof a therapeutically effective amount of the compound represented by Formula (I), a stereoisomer, a pharmaceutically acceptable salt, a pharmaceutically acceptable hydrate or solvate, or a pharmaceutically acceptable ester thereof as described in the first aspect of the present application, or the pharmaceutical composition as described in the third aspect of the present application, wherein the human pathological condition or symptom is improved by antagonizing A_{2A} adenosine receptor.

The fifth aspect of the present application provides the compound represented by Formula (I), a stereoisomer, a pharmaceutically acceptable salt, a pharmaceutically acceptable hydrate or solvate, or a pharmaceutically acceptable ester thereof as described in the first aspect of the present application for use in the prevention and/or treatment of a human pathological condition or symptom, wherein the human pathological condition or symptom is improved by antagonizing A_{2A} adenosine receptor.

The sixth aspect of the present application provides the compound represented by Formula (I), a stereoisomer, a pharmaceutically acceptable salt, a pharmaceutically acceptable hydrate or solvate, or a pharmaceutically acceptable ester thereof as described in the first aspect of the present application for use as an A_{2A} adenosine receptor antagonist.

According to some embodiments of the present application, the human pathological condition or symptom is selected from the group consisting of: ischemia, supraventricular arrhythmia, atrial fibrillation, acute renal failure, myocardial reperfusion injury, disease caused by fluid retention, allergic reaction, scleroderma, arthritis, inflammatory bowel disease, diabetes, obesity, Parkinson's disease, Huntington's disease, dystonia, dyskinesia, congestive heart failure, hypertension, dialysis hypotension, dementia, anxiety disorder, glaucoma, and alcoholism.

### Definition of substituents

The term "alkyl" as used herein refers to a saturated linear or branched monovalent hydrocarbonyl preferably having 1 to 6, 1 to 4 or 1 to 3 carbon atoms. For example, "C₁₋₆ alkyl" refers to a saturated linear or branched monovalent hydrocarbonyl having 1 to 6 carbon atoms. Typical examples of "alkyl" include, but are not limited to, methyl, ethyl, n-propyl, isopropyl, n-butyl, isobutyl, tert-butyl, n-pentyl, tert-pentyl, neopentyl, hexyl.

The term "hydroxyl" as used herein refers to -OH.

The term "halogen" as used herein refers to fluorine, chlorine, bromine or iodine. The preferred halogen is fluorine, chlorine or bromine.

The term "halogenated C₁₋₆ alkyl" as used herein refers to a C₁₋₆ alkyl mono- or polysubstituted by halogen such as fluorine, chlorine, bromine or iodine. The preferred halogenated alkyl is chloromethyl, chloroethyl, dichloroethyl, trifluoromethyl, difluoromethyl, monofluoromethyl, or the like.

The term "cycloalkyl" as used herein refers to a saturated cyclic hydrocarbonyl having 3 to 12 carbon atoms and having monocyclic or bicyclic or multiple rings (including fused and bridged ring systems), preferably having 3 to 10, 3 to 8, 5 to 8, 3 to 6 or 5 to 6 carbon atoms. Typical examples of "cycloalkyl" include, but are not limited to, monocyclic structures such as cyclopropyl, cyclobutyl, cyclopentyl, cyclohexyl, cycloheptyl, cyclooctyl, etc., bicyclic structures such as bicyclo[2.2.1]heptyl, and polycyclic structures such as adamantyl etc.

The term "heterocycloalkyl" as used herein refers to a cycloalkyl as defined herein that contains one, two or more heteroatoms independently selected from the group consisting of N, O and S. Typical examples of "heterocycloalkyl" include, but are not limited to, tetrahydrofuranyl, tetrahydrothienyl, pyrrolidinyl, piperazinyl, thiazinyl, piperidinyl, morpholinyl and the like.

The term "aryl" as used herein refers to an unsaturated aromatic carbocyclic group having 5 to 14 carbon atoms and having a monocyclic ring or fused ring of two or more rings. The aryl preferably has 5 to 10, 5 to 8 or 5 to 6 carbon atoms. Typical examples of "aryl" include, but are not limited to, phenyl, naphthyl, anthryl and the like.

The term "heteroaryl" as used herein refers to a heteroaromatic ring group having 5 to 14 ring members, including monocyclic heteroaromatic rings and polycyclic aromatic rings, in which one monocyclic aromatic ring is fused with one or more other aromatic rings. The heteroaryl has one or two or more heteroatoms selected from the group consisting of O, S and N. The term "heteroaryl" as used herein also includes groups in which an aromatic ring is fused with one or more non-aromatic rings (carbocyclic or heterocyclic rings), wherein the linking group or point is located on the aromatic ring or non-aromatic rings. The heteroaryl preferably has 5 to 10 ring members, more preferably 5 to 6 ring members. Typical examples of "heteroaryl" include, but are not limited to, furyl, imidazolyl, triazolyl, indolyl, thiazolyl, tetrazolyl, pyridyl, pteridyl, pyrimidinyl, triazolyl, quinolyl, isoquinolyl, quinazolinyl, quinoxalinyl and the like.

The term "C₁₋₆ alkoxy" as used herein refers to -OR¹¹, wherein R¹¹ is C₁₋₆ alkyl as defined herein. Typical examples of "C₁₋₆ alkoxy" include, but are not limited to, methoxy, ethoxy, n-propoxy, isopropoxy, n-butoxy, tert-butoxy, sec-butoxy, n-pentyloxy, n-hexyloxy, 1,2-dimethylbutoxy, etc.

When the name of the compound used herein is inconsistent with the chemical structural formula, the chemical structural formula shall prevail.

According to some embodiments of the present application, the pharmaceutically acceptable salt of the compound of Formula (I) as described in the present application includes salts formed with inorganic or organic acids, and salts formed with inorganic or organic bases. The present application relates to all forms of the above-mentioned salts, includes but not limited to: sodium salt, potassium salt, calcium salt, lithium salt, meglumine salt, hydrochloride, hydrobromide, hydriodate, nitrate, sulfate, hydrogen sulfate, phosphate, hydrogen phosphate, acetate, propionate, butyrate, oxalate, trimethylacetate, adipate, alginate, lactate, citrate, tartrate, succinate, maleate, fumarate, picrate, aspartate, gluconate, benzoate, methanesulfonate, ethanesulfonate, benzenesulfonate, p-toluenesulfonate and pamoate.

According to some embodiments of the present application, the compound of Formula (I) as described in the present application can form a pharmaceutically acceptable ester with an organic or inorganic acid. The pharmaceutically acceptable ester includes phosphate, sulfate, nitrate, formate, acetate, propionate, butyrate, valerate, and caproate, which are hydrolyzable in vivo.

The carrier described in the present application includes, but is not limited to: ion exchanger, alumina, aluminum stearate, lecithin, serum protein such as human albumin, buffer substance such as phosphate, glycerol, sorbic acid, potassium sorbate, mixture of partial glyceride of saturated plant fatty acid, water, salt or electrolyte such as protamine sulfate, disodium hydrogen phosphate, potassium hydrogen phosphate, sodium chloride, zinc salt, colloidal silica, magnesium trisilicate, polyvinylpyrrolidone, cellulosic substance, polyethylene glycol, sodium carboxymethylcellulose, polyacrylate, beeswax, lanolin.

The term "excipient" as used in the present application refers to an additive other than the main active ingredient in a pharmaceutical preparation. It is stable in nature, has no incompatibility with the main active ingredient, does not produce side effects, does not affect therapeutic effect, is not prone to deform, dry, crack, mildew, be worm-eaten at room temperature, is harmless to the human body, has no physiological effect, does not produce chemical or physical effects on the function of main active ingredient, does not affect the content determination of the main active ingredient, etc. For example, binder, filler, disintegrant, lubricant in tablet; preservative, antioxidant, corrigent, flavoring agent, cosolvent, emulsifier, solubilizer, osmotic pressure regulator and coloring agent in oral liquid preparation, etc. can all be called excipients.

The pharmaceutical composition described in the present application can be administered through various routes, such as oral tablet, capsule, powder, oral liquid, injection and transdermal preparation. The above-mentioned various preparation forms can be prepared according to conventional methods in the field of pharmacy. According to conventional pharmaceutical practices, the pharmaceutically acceptable carrier includes diluent, filler, disintegrant, wetting agent, lubricant, coloring agent, flavoring agent or other conventional additives. Typical pharmaceutically acceptable carriers include, for example, microcrystalline cellulose, starch, crospovidone, povidone, polyvinylpyrrolidone, maltitol, citric acid, sodium laurylsulfonate or magnesium stearate, etc.

According to the present application, the pharmaceutical composition can be administered in any of the following routes: oral administration, spray inhalation, rectal administration, nasal administration, buccal administration, vaginal administration, topical administration, parenteral administration such as subcutaneous, intravenous, intramuscular, intraperitoneal, intrathecal, intraventricular, intrasternal and intracranial injection or infusion, or administration with the aid of an explanted reservoir.

As described herein, "effective amount" refers to an amount that is sufficient to treat or prevent or diagnose a patient's disease but is sufficiently low to avoid serious side effects (at a reasonable benefit/risk ratio) within the scope of reasonable medical judgment. The therapeutically or prophylactically or diagnostically effective amount of the compound will vary according on the factors such as the specific compound selected (for example, considering the potency, effectiveness and half-life of the compound), the route of administration selected, the disease to be treated or prevented or diagnosed, the severity of the disease to be treated or prevented or diagnosed, the age, size, weight and physical disease of the patient being treated, the medical history of the patient to be treated, the duration of treatment or prevention or diagnosis, the nature of concurrent therapy, the desired effects of the treatment or prevention or diagnosis and so on, but it can still be routinely determined by those skilled in the art.

In addition, it should be noted that the specific dosage and usage of the compound of Formula (I) described in the present application for different patients depends on many factors, including the patient's age, weight, gender, natural health status, nutritional status, and the active strength of the compound, the administration time, metabolic rate, severity of disease and the subjective judgment of physician. Herein it is preferable to use a dose of 0.001 to 1000 mg/kg body weight/day.

### Beneficial technical effects of the present application

The compound represented by Formula (I), a stereoisomer, a pharmaceutically acceptable salt or hydrate provided in the present application can antagonize A_{2A} adenosine receptor, and thus can be used to prevent or treat a human pathological condition or symptom, wherein the human pathological condition or symptom can be improved by antagonizing the activity of A_{2A} adenosine receptor.

### Specific Models for Carrying Out the present application

The present application can be further described through the following examples and test examples. However, the scope of the present application is not limited to the following examples or test examples. Those skilled in the art can understand that various changes and modifications can be made to the present application without departing from the spirit and scope of the present application. The present application provides a general and/or specific description of the materials and test methods used in the test. Although many materials and operating methods used to achieve the purpose of the present application are well known in the art, the present application is still described herein as much detail as possible.

For all the following examples, standard operations and purification methods known to those skilled in the art could be used. Unless otherwise stated, all temperatures were expressed in °C (Celsius). The structure of the compound was determined by nuclear magnetic resonance (NMR) or mass spectrometry (MS). The compound's melting point m.p. is determined by RY-1 melting point meter, in which the thermometer had not been corrected, and the m.p. was given in °C. 1H NMR was measured by JEOL JNM-ECA-400 NMR spectrometer. The mass spectrum was measured by API3000 (ESI) instrument. All solvents in reaction that were not specified were subject to standardized pretreatment.

### Example 1: Synthesis of N-{4-{7-[(2R,3R,4S,5R)-3,4-dihydroxy-5-(hydroxymethyl) tetrahydrofuran-2-yl]-7H-[1,2,4]triazolo[3,4-i]purin-3-yl}phenyl}acetamide (Compound 1)

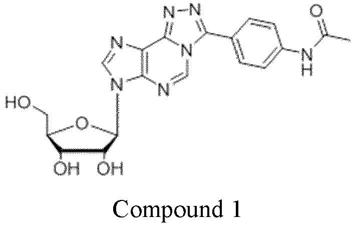

### 1.1 Synthesis of (2R,3R,4S,5R)-2-(6-hydrazino-9H-purin-9-yl)-5-(hydroxymethyl) tetrahydrofuran-3,4-diol (Compound of Formula II)

5g (0.018mol) of (2R,3R,4S,5R)-2-(6-chloro-9H-punn-9-yl)-5-(hydroxymethyl) tetrahydrofuran-3,4-diol (Compound of Formula V) was added to 10ml of hydrazine hydrate (65wt% aqueous solution), heated to 70°C under stirring, continuously heated for 2 hours until the reactant (Compound of Formula V) disappeared, the progress of the reaction was monitored by TLC (CH₂Cl₂:MeOH = 3:1 (v/v)). Then the reaction mixture was heated to 25°C, and 2-propanol (50ml) was added for dilution under stirring overnight. The separated precipitate was filtered to obtain 4.8 g of (2R,3R,4S,5R)-2-(6-hydrazino-9H-purin-9-yl)-5-(hydroxymethyl)tetrahydrofuran-3,4-diol (Compound of Formula II) as a white solid, which was directly used in the next reaction.

### 1.2 Synthesis of N-{4-{(E)-{2-{9-[(2R,3R,4S,5R)-3,4-dihydroxy-5-(hydroxymethyl) tetrahydrofuran-2-yl]-9H-purin-6-yl}hydrazono}methyl}phenyl}acetamide (Compound of Formula IV)

0.5g (0.0018mol) of (2R,3R,4S,5R)-2-(6-hydrazino-9H-purin-9-yl)-5-(hydroxymethyl) tetrahydrofuran-3,4-diol (Compound of Formula II) and 0.33g (0.002mol) of 4-acetamidobenzaldehyde (Compound of Formula III, wherein R₁ was 4-acetamidophenyl) (1.1 equivalents) were mixed in methanol (20ml) and heated by microwave at 70°C for 30 minutes. The crude product was precipitated from methanol. After filtration, the crude product was further purified to obtain 323 mg of white solid (Compound of Formula IV), which was directly used in the next reaction.

### 1.3 Synthesis of N-{4-{7-[(2R,3R,4S,5R)-3,4-dihydroxy-5-(hydroxymethyl) tetrahydrofuran-2-yl]-7H-[1,2,4]triazolo[3,4-i]purin-3-yl}phenyl}acetamide (Compound 1)

0.5g (0.0012mol) of N-{4-{(E)-{2-{9-[(2R,3R,4S,5R)-3,4-dihydroxy-5-(hydroxymethyl) tetrahydrofuran-2-yl]-9H-purin-6-yl}hydrazono}methyl}phenyl}acetamide (Compound of Formula IV) was added to 10ml of acetic acid and stirred to dissolve, then 0.4g (0.0024mol) of NBS (N-bromosuccinimide) (2.0 equivalents) was slowly added in batch, continuously stirred for 1 hour. The resulting solution was distilled under reduced pressure to remove acetic acid, and the residue was purified by flash chromatography, in which a mixed solvent of CH₂Cl₂ and MeOH (CH₂Cl₂:MeOH = 10:1 (v /v)) was used as the eluent, and 224 mg of white solid (Compound 1) was obtained. m.p. 132°C; ¹H NMR (DMSO-*d*₆): δ (ppm) 10.26(s, 1H), 9.26(s, 1H), 8.70(s, 1H), 7.93(d, 2H J=8.4Hz), 7.85(d, 2H J=8.4Hz),6.08(d, 1H, J=5.6Hz), 5.58(d, 1H, J=6.0Hz), 5.28(d, 1H, J=4.8Hz), 5.08(t, 1H, J=5.6Hz), 4.20(dd, 1H, J=4.0Hz,4.8Hz), 4.00(dd, 1H, J=4.0Hz,4.0Hz), 3.74-3.59(m, 2H), 2.12(s, 3H); HRMS (ESI+) m/z [M + H]⁺ calculated for C₁₉H₁₉N₇O₅: 426.1520; found: 426.1520.

The following Compounds 2 to 13 could be prepared by referring to the method of Example 1, using different reactants (various substituted formaldehydes represented by Formula III) instead of 4-acetamidobenzaldehyde in step 1.2.

### Example 2: Synthesis of (2R,3R,4S,5R)-2-{3-cyclopentyl-7H-[1,2,4]triazolo[3,4-i] purin-7-yl}-5-(hydroxymethyl)tetrahydrofuran-3,4-diol (Compound 2)

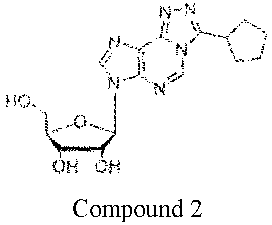

Referring to the method of steps 1.2 and 1.3 in Example 1, replacing the 4-acetamidobenzaldehyde in step 1.2 with cyclopentanecarbaldehyde, the title compound was obtained as 152 mg of white solid (Compound 2). m.p. 84°C; ¹H NMR (DMSO-*d*₆): δ (ppm) 9.30(s, 1H), 8.66(s, 1H), 6.06(d, 1H J=5.6Hz), 5.60(br, 1H), 5.33(br, 1H), 5.11(br, 1H), 4.57(t, 1H, J=5.2Hz), 4.19(t, 1H, J=4.0Hz), 4.00(dd, 1H, J=4.0Hz,3.6Hz), 3.81-3.72(m, 1H), 3.73-3.58(m, 2H), 2.24-2.18(m, 2H), 2.03-1.93(m, 2H), 1.80-1.68(m, 4H); HRMS (ESI+) m/z [M + H]⁺ calculated for C₁₆H₂₀N₆O₄: 361.1619; found: 361.1619.

### Example 3: Synthesis of (2R,3R,4S,5R)-2-{3-[3,4-di(benzyloxy)phenyl]-7H-[1,2,4] triazolo[3,4-i]purin-7-yl}-5-(hydroxymethyl)tetrahydrofuran-3,4-diol (Compound 3)

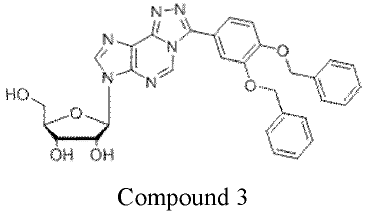

Referring to the method of steps 1.2 and 1.3 in Example 1, replacing the 4-acetamidobenzaldehyde in step 1.2 with 3,4-benzyloxybenzaldehyde, the title compound was obtained as 190 mg of white solid (Compound 3). m.p. 116°C; ¹H NMR (DMSO-*d*₆): δ (ppm) 9.13(s, 1H), 8.72(s, 1H), 7.68(d, 1H J=1.6Hz), 7.52-7.30(m, 12H), 6.09(d, 1H, J=5.2Hz), 5.61(d, 1H, J=5.6), 5.33(d, 1H, J=5.2Hz),5.27(s, 4H), 5.11(t, 1H, J=5.2Hz), 4.59(dd, 1H, J=5.6Hz, 5.2Hz), 4.21(dd, 1H J=4.0Hz, 4.4Hz), 4.01(dd, 1H, J=3.6Hz,3.6Hz), 3.75-3.58(m, 2H); HRMS (ESI+) m/z [M + H]⁺ calculated for C₃₁H₂₈N₆O₆: 581.2143; found: 581.2141.

### Example 4: Synthesis of (2R,3R,4S,5R)-2-{3-{4-[(4-fluorobenzyl)oxy]phenyl}-7H-[1,2,4] triazolo[3,4-i]purin-7-yl}-5-(hydroxymethyl)tetrahydrofuran-3,4-diol (Compound 4)

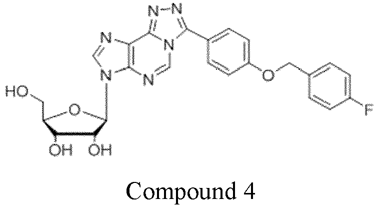

Referring to the method of steps 1.2 and 1.3 in Example 1, replacing the 4-acetamidobenzaldehyde in step 1.2 with 4-(4-fluorobenzyloxy)benzaldehyde, the title compound was obtained as 235 mg of white solid (Compound 4). m.p. 112°C; ¹H NMR (DMSO-*d*₆): δ (ppm) 9.21(s, 1H), 8.71(s, 1H), 7.93(d, 2H, J=8.8Hz), 7.56(t, 2H, J=8.8Hz), 7.26(t, 4H, J=8.8Hz), 6.08(d, 1H, J=5.6Hz), 5.61(d, 1H, J=5.6), 5.32(d, 1H, J=4.8Hz), 5.22(s, 2H), 5.10(t, 1H, J=5.6Hz), 4.58(d, 1H, J=4.8Hz), 4.20(d, 1H J=4.0Hz), 4.00(d, 1H, J=4.0Hz), 3.74-3.57(m, 2H); HRMS (ESI+) m/z [M + H]⁺ calculated for C₂₄H₂₁FN₆O₅: 493.1630; found: 493.1630.

### Example 5: Synthesis of (2R,3R,4S,5R)-2-{3-[3-(benzyloxy)phenyl]-7H-[1,2,4] triazolo[3,4-i]purin-7-yl}-5-(hydroxymethyl)tetrahydrofuran-3,4-diol (Compound 5)

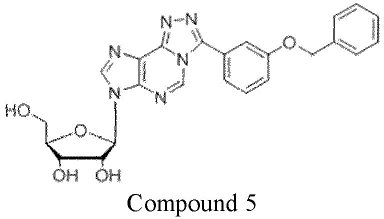

Referring to the method of steps 1.2 and 1.3 in Example 1, replacing the 4-acetamidobenzaldehyde in step 1.2 with 3-benzyloxybenzaldehyde, the title compound was obtained as 220 mg of white solid (Compound 5). m.p. 76°C; ¹H NMR (DMSO-*d*₆): δ (ppm) 9.22(s, 1H), 8.73(s, 1H), 7.64-7.27(m, 9H), 6.08(d, 1H, J=5.2Hz), 5.61(d, 1H, J=6.0Hz), 5.32(d, 1H, J=5.2Hz), 5.25(s, 2H), 5.11(t, 1H, J=5.6Hz), 4.59(dd, 1H, J=5.6Hz, 5.2Hz), 4.20(dd, 1H J=4.4Hz, 4.4Hz), 4.00(dd, 1H, J=4.0Hz, 3.6Hz), 3.74-3.57(m, 2H); HRMS (ESI+) m/z [M + H]⁺ calculated for C₂₄H₂₂N₆O₅: 475.1724; found: 475.1724.

### Example 6: Synthesis of (2R,3R,4S,5R)-2-{3-[2,4-di(trifluoromethyl)phenyl]-7H-[1,2,4] triazolo[3,4-i]purin-7-yl}-5-(hydroxymethyl)tetrahydrofuran-3,4-diol (Compound 6)

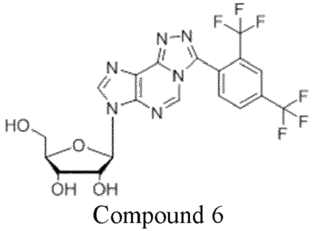

Referring to the method of steps 1.2 and 1.3 in Example 1, replacing the 4-acetamidobenzaldehyde in step 1.2 with 2,4-di(trifluoromethyl)benzaldehyde, the title compound was obtained as 205mg of white solid (Compound 6). ¹H NMR (DMSO-*d*₆): δ (ppm) 9.10(s, 1H), 8.77(s, 1H), 8.41-8.24(m, 3H), 6.08(d, 1H, J=5.6Hz), 5.60(d, 1H, J=6.0), 5.31(d, 1H, J=4.4Hz), 5.11(t, 1H, J=5.2Hz), 4.58(dd, 1H, J=5.6Hz, 5.2Hz), 4.19(d, 1H J=4.0Hz), 4.00(d, 1H, J=4.0Hz), 3.73-3.57(m, 2H); HRMS (ESI+) m/z [M + H]⁺ calculated for C₁₉H₁₄F₆N₆O₄: 505.1053; found: 505.1052.

### Example 7: Synthesis of (2R,3S,4R,5R)-2-{3-[4-(pyridin-2-yl)phenyl]-7H-[1,2,4] triazolo[3,4-i]purin-7-yl}-5-(hydroxymethyl)tetrahydrofuran-3,4-diol (Compound 7)

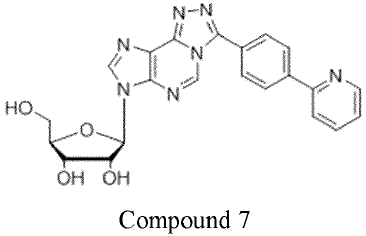

Referring to the method of steps 1.2 and 1.3 in Example 1, replacing the 4-acetaminobenzaldehyde in step 1.2 with 4-(2-pyridyl)benzaldehyde, the title compound was obtained as 195 mg of white solid (Compound 7). m.p. 172°C; ¹H NMR (DMSO-*d*₆): δ (ppm) 9.37(s, 1H), 8.75(s, 2H), 8.37(d, 2H, J=8.4Hz), 8.15-8.12(m, 3H), 7.99-7.95(m, 1H), 7.46-7.43(m, 1H), 6.10(d, 1H, J=5.6Hz), 5.66(br,1H), 5.38(br, 1H), 5.14(br, 1H), 4.61(t, 1H, J=5.2Hz), 4.22(t, 1H, J=4.0Hz), 4.20(d, 1H, J=4.0Hz), 3.74-3.59(m, 2H); HRMS (ESI+) m/z [M + H]⁺ calculated for C₂₂H₁₉N₇O₄: 446.1571; found: 446.1571.

### Example 8: Synthesis of (2R,3R,4S,5R)-2-{3-{[1,1'-biphenyl]-4-yl}-7H-[1,2,4]triazolo[3, 4-i]purin-7-yl}-5-(hydroxymethyl)tetrahydrofuran-3,4-diol (Compound 8)

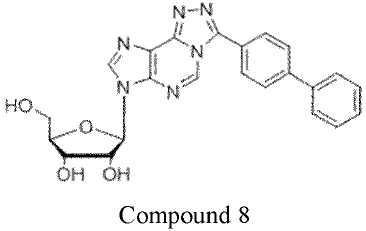

Referring to the method of steps 1.2 and 1.3 in Example 1, replacing the 4-acetamidobenzaldehyde in step 1.2 with 4-biphenylaldehyde, the title compound was obtained as 175 mg of white solid (Compound 8). m.p. 280°C; ¹H NMR (DMSO-*d*₆): δ (ppm) 9.34(s, 1H), 8.74(s, 1H), 8.10(d, 2H, J=8.4Hz), 7.96(d, 2H, J=8.4Hz), 7.81(d, 2H, J=7.2Hz), 7.55(t, 2H, J=7.2Hz), 7.46(t, 1H, J=7.2Hz), 6.10(d, 1H, J=5.6Hz), 5.62(d, 1H, J=5.6Hz), 5.33(d, 1H, J=4.8Hz), 5.13(t, 1H, J=5.6Hz), 4.60(d, 1H, J=5.2Hz), 4.21(d, 1H J=4.0Hz), 4.01(dd, 1H, J=4.0Hz, 3.6Hz), 3.74-3.58(m, 2H); HRMS (ESI+) m/z [M + H]⁺ calculated for C₂₃H₂₀N₆O₄: 445.1619; found: 445.1619.

### Example 9: Synthesis of (2R,3S,4R,5R)-2-{3-(4-propoxyphenyl)-7H-[1,2,4]triazolo[3,4-i]purin-7-yl}-5-(hydroxymethyl)tetrahydrofuran-3,4-diol (Compound 9)

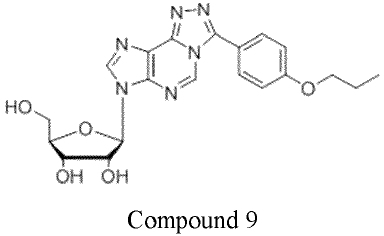

Referring to the method of steps 1.2 and 1.3 in Example 1, replacing the 4-acetaminobenzaldehyde in step 1.2 with 4-propoxybenzaldehyde, the title compound was obtained as 185 mg of white solid (Compound 9). m.p. 118°C; ¹H NMR (DMSO-*d*₆): δ (ppm) 9.21(s, 1H), 8.71(s, 1H), 7.90(d, 2H, J=8.8Hz), 7.18(d, 2H, J=8.8Hz), 6.08(d, 1H, J=5.6Hz), 5.62(d, 1H, J=6.0Hz), 5.32(d, 1H, J=5.2Hz), 5.12(t, 1H, J=5.2Hz), 4.58(dd, 1H, J=5.2Hz, 5.2Hz), 4.20(d, 1H J=3.6Hz),4.06(t, 2H, J=6.8Hz), 4.00(d, 1H, J=3.6Hz), 3.74-3.58(m, 2H), 1.83-1.75(m, 2H), 1.03(t, 3H, J=7.6Hz); HRMS (ESI+) m/z [M + H]⁺ calculated for C₂₀H₂₂N₆O₅: 427.1724; found: 427.1725.

### Example 10: Synthesis of (2R,3S,4R,5R)-2-{3-[4-(trifluoromethyl)phenyl]-7H-[1,2,4] triazolo[3,4-i]purin-7-yl}-5-(hydroxymethyl)tetrahydrofuran-3,4-diol (Compound 10)

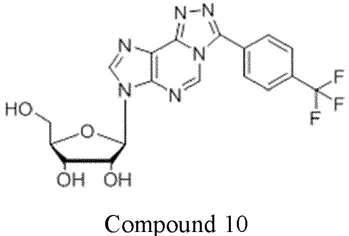

Referring to the method of steps 1.2 and 1.3 in Example 1, replacing the 4-acetamidobenzaldehyde in step 1.2 with 4-(trifluoromethyl)benzaldehyde, the title compound was obtained as 165 mg of white solid (Compound 10). m.p. 124°C; ¹H NMR (DMSO-*d*₆): δ (ppm) 9.37(s, 1H), 8.75(s, 1H), 8.25(d, 2H, J=8.0Hz), 8.02(d, 2H, J=8.0Hz), 6.10(d, 1H, J=5.6Hz), 5.62(d, 1H, J=6.0Hz), 5.33(d, 1H, J=4.8Hz), 5.12(t, 1H, J=5.2Hz), 4.60(dd, 1H, J=5.2Hz, 5.6Hz), 4.21(dd, 1H J=3.6Hz, 4.8Hz), 4.01(d, 1H, J=4.0Hz), 3.75-3.58(m, 2H); HRMS (ESI+) m/z [M + H]⁺ calculated for C₁₈H₁₆F₃N₆O₄: 437.1180; found: 437.1179.

### Example 11: Synthesis of (2R,3R,4S,5R)-2-{3-(5-bromopyridin-2-yl)-7H-[1,2,4] triazolo[3,4-i]purin-7-yl}-5-(hydroxymethyl)tetrahydrofuran-3,4-diol (Compound 11)

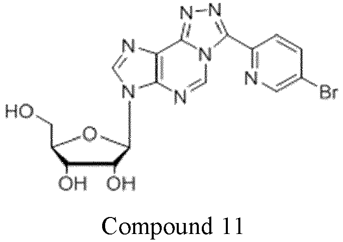

Referring to the method of steps 1.2 and 1.3 in Example 1, replacing the 4-acetamidobenzaldehyde in step 1.2 with 5-bromo-2-pyridinecarbaldehyde, the title compound was obtained as 155 mg of yellow solid (Compound 11). m.p. 222°C; ¹H NMR (DMSO-*d*₆): δ (ppm) 10.29(s, 1H), 8.97(s, 1H), 8.77(s, 1H), 8.38-8.32(m, 2H), 6.11(d, 1H, J=5.6Hz), 5.64(d, 1H, J=6.0Hz), 5.33(d, 1H, J=5.2Hz), 5.12(t, 1H, J=5.6Hz), 4.63(dd, 1H, J=5.2Hz, 5.6Hz), 4.23(dd, 1H J=4.0Hz, 4.8Hz), 4.02(dd, 1H, J=4.0Hz, 3.6Hz), 3.76-3.59(m, 2H); HRMS (ESI+) m/z [M + H]⁺ calculated for C₁₆H₁₄BrN₇O₄: 448.0363; found: 448.0361.

### Example 12: Synthesis of (2R,3S,4R,5R)-2-{3-(thiazol-5-yl)-7H-[1,2,4]triazolo[3,4-i] purin-7-yl}-5-(hydroxymethyl)tetrahydrofuran-3,4-diol (Compound 12)

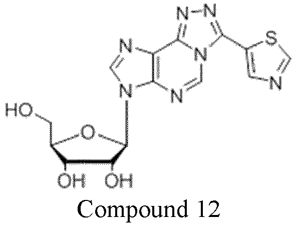

Referring to the method of steps 1.2 and 1.3 in Example 1, replacing the 4-acetamidobenzaldehyde in step 1.2 with thiazole-5-carbaldehyde, the title compound was obtained as 190 mg of white solid (Compound 12). m.p. 216°C; ¹H NMR (DMSO-*d*₆): δ (ppm) 9.52(s, 1H), 9.39(s, 1H), 8.86(s, 1H), 8.76(s, 1H), 6.10(d, 1H, J=5.6Hz), 5.64(d, 1H, J=5.6Hz), 5.34(d, 1H, J=4.8Hz), 5.12(s, 1H), 4.59(dd, 1H, J=5.2Hz, 5.6Hz), 4.21(d, 1H J=4.0Hz), 4.01(dd, 1H, J=4.0Hz, 4.0Hz), 3.75-3.59(m, 2H); HRMS (ESI+) m/z [M + H]⁺ calculated for C₁₄H₁₃N₇O₄S: 376.0822; found: 376.0820.

### Example 13: Synthesis of (2R,3R,4S,5R)-2-{3-cyclohexyl-7H-[1,2,4]triazolo[3,4-i] purin-7-yl}-5-(hydroxymethyl)tetrahydrofuran-3,4-diol (Compound 13)

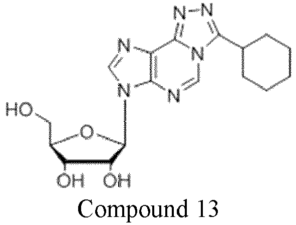

Referring to the method of steps 1.2 and 1.3 in Example 1, replacing the 4-acetamidobenzaldehyde in step 1.2 with cyclohexanecarbaldehyde, the title compound was obtained as 215 mg of white solid (Compound 13). m.p. 130°C; ¹H NMR (DMSO-*d*₆): δ (ppm) 9.37(s, 1H), 8.65(s, 1H), 6.06(d, 1H, J=5.6Hz), 5.60(br, 1H), 5.32(br, 1H), 5.10(s, 1H), 4.57(t, 1H, J=5.2Hz), 4.19(d, 1H J=4.4Hz), 4.00(dd, 1H, J=3.6Hz, 4.0Hz), 3.72-3.58(m, 2H), 2.09-1.31(m, 10H); HRMS (ESI+) m/z [M + H]⁺ calculated for C₁₇H₂₂N₆O₄: 375.1775; found: 375.1776.

### Example 14: Radioligand binding test

### 1) Experimental materials

[3H] CGS21680(2-[p-(2-carboxyethyl)phenylethylamino]-5'-N-ethylcarboxamidoadenosine, [carboxy-1-ethyl-3H(N)]-; 250µCi) was purchased from PerkinElmer Research Products (Boston, MA).

Cell membranes stably transfected with (human) A_{2A} adenosine receptor were prepared in HEK-293 cells. The cell membranes were obtained from PerkinElmer Research Products (Boston, MA).

CGS 21680 (2-[p-(2-carboxyethyl)phenylethylamino]-5'-ethylcarboxamidoadenosine) was purchased from Selleck (Shanghai, CN).

All other reagents were of analytical grade and obtained from commercial sources.

### 2) Experimental method

The A_{2A} adenosine receptors used were all expressed in the cell membranes. The compound was diluted 3 times serially with DMSO (Solarbio, D8371-250ml) to generate compound source plates with 10 different concentrations (10µM, 3.3µM, 1.1µM, 0.37µM, 0.12µM, 0.0412µM, 0.0137µM, 0.0046µM, 0.0015µM, 0.0005µM), 250 nL of compound was added to 384-well Opti-plate, sealed with parafilm. 20 U hA_{2A} HEK-293 cell membrane was diluted with 1 mL of assay buffer (50 mM Tris-HCl, pH 7.4, 10 mM MgCl_{2,} 1 mM EDTA, 1µg/mL adenosine deaminase), 0.75 uCi [3H]-CGS 21680 (final 25 nM) was added to the diluted cell membrane and mixed well. 50 µL of the prepared dilution solution of cell membrane was transferred to the 384-well Opti-plate containing compound and incubated at 25°C for 90 minutes. 100 µL of 0.5% Polyethyleneimine solution (PEI) was added to UNIFILTER-96 GF/B filter plate, and soaked for 90 minutes at 4°C, then 500 µL of washing buffer/well (50mM Tris-HCl, pH 7.4, 154mM NaCl) was transferred with Cell Harvester to wash the UNIFILTER-96 GF/B filter plate twice. The mixed system in the Opti-plate was transferred to the washed UNIFILTER-96 GF/B filter plate, 500 µL of washing buffer/well (50mM Tris-HCl, pH 7.4, 154mM NaCl) was used to wash the UNIFILTER-96 GF/B filter plate 9 times. Incubation was performed at 37°C for 3 minutes. 40 µL of ULTIMA GOLD scintillation solution (Perkin Elmer, Cat #77-16061) was added to each well, CPM (count per minute) value was read by a MicroBeta liquid scintillation counter (PerkinElmer). The specific binding percentage of [3H]CGS21680 was calculated according to the CPM value, % specific binding of [3H]CGS21680 = (CPMₛₐₘₚₗₑ - CPM_{Low Control}) / (CPM_{High Control} - CPM_{Low Control}) ^{∗} 100, in which High Control was 0.5% DMSO, Low Control was 100 µM CGS21680. The IC₅₀ value was calculated by curve fitting according to the compound concentration and the specific binding percentage of [3H]CGS21680.

### 3) Experimental results

The inhibition constant (Kᵢ) value was calculated from the IC₅₀ value according to the Cheng and Prusoff equation, Kᵢ= IC₅₀/ (1+[S]/Kₘ), wherein [S] was the concentration of radioligand (25nM) and Kₘ was dissociation constant (22 nM) of [3H] CGS21680 binding to human A_{2A}AR. Table 1 showed the inhibition constant Ki for Compounds 1 to 13 of the present application binding to A_{2A} adenosine receptor.

**Table 1: Test results of compounds binding to A_{2A} adenosine receptor**

| Compound | IC₅₀(nM) | K*ᵢ* (nM) |
|---|---|---|
| Compound 1 | 6.4 | 3.0 |
| Compound 2 | 3.9 | 1.8 |
| Compound 3 | >10,000 | >10,000 |
| Compound 4 | 3.9 | 1.8 |
| Compound 5 | 6.2 | 2.9 |
| Compound 6 | >10,000 | >10,000 |
| Compound 7 | >10,000 | >10,000 |
| Compound 8 | 7509 | 3515 |
| Compound 9 | 4.3 | 2.0 |
| Compound 10 | >10,000 | >10,000 |
| Compound 11 | >10,000 | >10,000 |
| Compound 12 | 3.9 | 1.8 |
| Compound 13 | >10,000 | >10,000 |

### Example 15: A_{2A} adenosine receptor cAMP test

### 1) Experimental materials

Experimental reagents and consumables: DMEM/F12, G418, Penicillin-Streptomycin, Versene Solution, HEPES, Hank's Buffered Saline Solution, PBS (pH 7.4, 1×, sterile), FBS, BSA Stabilizer 7.5%, Rolipram, NECA were separately purchased from Gibico, Hyclone and Sigma. LANCE^{®} Ultra cAMP kit (Eu-cAMP tracer, Ulight-anti-cAMP reagent, cAMP detection buffer) and hADORA_{2A}-HEK293 cells were purchased from PerkinElmer Research Products (Boston, MA). All other reagents were of analytical grade and obtained from commercial sources. 384-well polypropylene microplate and 384-well solid white plate were purchased from Labcyte and Corning, respectively.

Experimental instruments: TECAN automated liquid handling workstation, Echo Acoustic Liquid Handler and EnVison multimode plate reader were purchased from TECAN, Labcyte and Envision, respectively.

### 2) Experimental method

The cells stably expressing human adenosine receptor A_{2A} (hADORA_{2A}-HEK293 cells) were cultured in DMEM/F12 medium containing 10% FBS, 1× Penicillin-Streptomycin and 400ug/ml G418 in a 37°C and 5% CO₂ environment. Before the experiment, the cells were digested with Versene solution, and the cells were collected by centrifugation at 200g and room temperature for 5 minutes, and finally resuspended with assay buffer (Hank's buffered saline solution, containing 5mM HEPES, 0.1% BSA stabilizer and 10µM Rolipram, pH 7.4). The TECAN automated liquid handling workstation was used to dilute the compound in a 384-well polypropylene microplate with DMSO to 11 concentration points in a 3-fold gradient to prepare the compound source plate, in which the 11 concentration points of the compound were 10mM, 3.33mM, 1.11mM., 0.37mM, 0.12mM, 0.041mM, 0.013mM, 4.57×10⁻³mM, 1.52×10⁻³mM, 5×10⁻⁴mM and 1.7×10⁻⁴mM. The Echo Acoustic Liquid Handler (Labcyte) was used to transfer the test compound from the compound source plate to an assay plate, in which the transfer volume of the compound was 10 nl/well. The hADORA_{2A}-HEK293 cell suspension was diluted with assay buffer to 30,000 cells/ml, and the cell suspension was transferred to the assay plate at a volume of 10 µl/well (300 cells/well). The assay plate was centrifuged at 150g for 1 minute and pre-incubated at room temperature for 30 minutes. The Eu-cAMP tracer working solution (Eu-cAMP tracer 40µl, cAMP detection buffer 1.96ml) (5µl/well) was added to the assay plate, and then the Ulight-anti-cAMP working solution (13µl of Ulight-anti-cAMP reagent and 1.95ml of cAMP detection buffer) (5µl/well) was added to the assay plate. The assay plate was rotated at 150g for 30 seconds and incubated at room temperature for 30 minutes. The EnVison multimode plate reader (PerkinElmer) was used determine the level of cyclic adenosine monophosphate in the final solution (λₑₓ=320nm, λₑₘ=665nm & 615nm). The EC₅₀ (nM) value of the compound interacting with the A_{2A} adenosine receptor to stimulate the production of cyclic adenosine monophosphate was calculated and the titer of the compound function was expressed as the EC₅₀ (nM) value.

### 3) Experimental results

When the test compounds interacted with A_{2A}AR, the EC₅₀(nM) values for stimulating the production of cyclic AMP were shown in Table 2. The results showed that Compounds 1, 2, 4, 5, 9, 12 prepared in the present application were all shown as hA_{2A}AR antagonists. The cAMP EC₅₀ values of Compounds 1, 2, 4, 5, 9, 12 were basically all greater than 10 µM, which could not stimulate the production of cyclic adenosine monophosphate, indicating these compounds had no agonistic activity.

**Table 2: EC₅₀ value test results of compound A_{2A} antagonist function determination**

| Compound | cAMP EC₅₀ (nM) |
|---|---|
| Compound 1 | > 10000 |
| Compound 2 | > 10000 |
| Compound 4 | > 10000 |
| Compound 5 | > 10000 |
| Compound 9 | > 10000 |
| Compound 12 | > 10000 |

Although the specific embodiments of the present application have been described in detail, those skilled in the art will understand that according to all the teachings that have been disclosed, various modifications and substitutions can be made to those details, and these changes are all within the protection scope of the present application. The full scope of the present application is given by the appended claims and any equivalents thereof. The publications, patents and patent documents cited in the present application are incorporated herein by reference.

## Claims

1. A compound represented by Formula (I), a stereoisomer, a pharmaceutically acceptable salt, a pharmaceutically acceptable hydrate or solvate, or a pharmaceutically acceptable ester thereof, 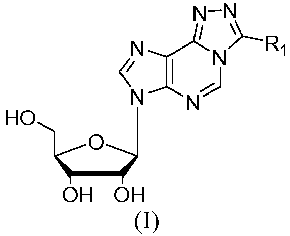 wherein:
R₁ is selected from the group consisting of aryl, cycloalkyl, heteroaryl, heterocycloalkyl, aryl substituted by one or more substituents R¹⁰, cycloalkyl substituted by one or more substituents R¹⁰, heteroaryl substituted by one or more substituents R¹⁰, heterocycloalkyl substituted by one or more substituents R¹⁰, wherein R¹⁰ is selected from the group consisting of halogen, C₁₋₆ alkoxy, C₁₋₆ alkyl, hydroxyl, amino, cyano, nitro, halogenated C₁₋₆ alkyl, -NHC(O)R¹¹, benzyloxy, halogenated phenylmethoxy, aryl, heteroaryl, R¹¹ is C₁₋₆ alkyl (e.g., methyl, ethyl, n-propyl, isopropyl, n-butyl, isobutyl, tert-butyl, n-pentyl, tert-pentyl, neopentyl, hexyl).

2. The compound, a stereoisomer, a pharmaceutically acceptable salt, a pharmaceutically acceptable hydrate or solvate, or a pharmaceutically acceptable ester thereof according to claim 1, wherein R₁ is selected from the group consisting of phenyl, cyclopentyl, cyclohexyl, pyridyl, thiazolyl, pyrrolyl, imidazolyl, furyl, phenyl substituted by one or more substituents R¹⁰, cyclopentyl substituted by one or more substituents R¹⁰, cyclohexyl substituted by one or more substituents R¹⁰, pyridyl substituted by one or more substituents R¹⁰, thiazolyl substituted by one or more substituents R¹⁰, pyrrolyl substituted by one or more substituents R¹⁰, imidazolyl substituted by one or more substituents R¹⁰, and furanyl substituted by one or more substituents R¹⁰.

3. The compound, a stereoisomer, a pharmaceutically acceptable salt, a pharmaceutically acceptable hydrate or solvate, or a pharmaceutically acceptable ester thereof according to claim 1, wherein R₁ is selected from the group consisting of phenyl, cyclopentyl, cyclohexyl, pyridin-2-yl, thiazol-5-yl, phenyl substituted by one or more substituents R¹⁰, cyclopentyl substituted by one or more substituents R¹⁰, cyclohexyl substituted by one or more substituents R¹⁰, pyridin-2-yl substituted by one or more substituents R¹⁰, and thiazol-5-yl substituted by one or more substituents R¹⁰.

4. The compound, a stereoisomer, a pharmaceutically acceptable salt, a pharmaceutically acceptable hydrate or solvate, or a pharmaceutically acceptable ester thereof according to any one of claims 1 to 3, wherein R¹⁰ is selected from the group consisting of halogen, C₁₋₄ alkoxy, C₁₋₄ alkyl, hydroxyl, amino, cyano, nitro, halogenated C₁₋₄ alkyl, -NHC(O)R¹¹, benzyloxy, halogenated phenylmethoxy, aryl, and heteroaryl, R¹¹ is C₁₋₄ alkyl, or
R¹⁰ is selected from the group consisting of fluorine, chlorine, bromine, iodine, methyl, ethyl, n-propyl, isopropyl, n-butyl, isobutyl, tert-butyl, n-pentyl, tert-pentyl, neopentyl, hexyl, trifluoromethyl, difluoromethyl, fluoromethyl, methoxy, ethoxy, n-propoxy, isopropoxy, n-butoxy, tert-butoxy, sec-butoxy, n-pentyloxy, n-hexyloxy, hydroxyl, amino, cyano, nitro, acetamido, formylamino, propionamido, benzyloxy, fluorophenylmethoxy, phenyl, pyridyl, pyrrolidinyl, cyclopentyl, cyclohexyl, morpholinyl, imidazolyl, and thiazolyl, or
R¹⁰ is selected from the group consisting of fluorine, chlorine, bromine, methyl, ethyl, n-propyl, isopropyl, n-butyl, isobutyl, tert-butyl, trifluoromethyl, methoxy, ethoxy, n-propoxy, isopropoxy, n-butoxy, tert-butoxy, sec-butoxy, hydroxyl, amino, cyano, nitro, acetamido, formylamino, propionamido, benzyloxy, 4-fluorophenylmethoxy, phenyl, pyridin-2-yl, 5-bromopyridin-2-yl, cyclopentyl, and cyclohexyl.

5. The compound, a stereoisomer, a pharmaceutically acceptable salt, a pharmaceutically acceptable hydrate or solvate, or a pharmaceutically acceptable ester thereof according to any one of claims 1 to 3, wherein R₁ is 4-acetamidophenyl, cyclopentyl, 3,4-dibenzyloxyphenyl, 4-(4-fluorobenzyloxy)phenyl, 3-benzyloxyphenyl, 2,4-di(trifluoromethyl)phenyl, 4-(pyridin-2-yl)phenyl, 4-phenylphenyl, 4-propoxyphenyl, 4-trifluoromethylphenyl, 5-bromopyridin-2-yl, thiazol-5-yl or cyclohexyl.

6. The compound, a stereoisomer, a pharmaceutically acceptable salt, a pharmaceutically acceptable hydrate or solvate, or a pharmaceutically acceptable ester thereof according to any one of claims 1 to 3, wherein R₁ is phenyl or phenyl substituted by one or more substituents R¹⁰, R¹⁰ is selected from the group consisting of halogen, -NHC(O)R¹¹, C₁₋₆ alkoxy, C₁₋₆ alkyl, hydroxyl, amino, cyano, nitro, halogenated C₁₋₆ alkyl, benzyloxy, halogenated phenylmethoxy, aryl, and heteroaryl, R¹¹ is C₁₋₆ alkyl (e.g., methyl, ethyl, n-propyl, isopropyl, n-butyl, isobutyl, tert-butyl, n-pentyl, tert-pentyl, neopentyl, hexyl),
for example, R¹⁰ is selected from the group consisting of halogen, C₁₋₄ alkoxy, C₁₋₄ alkyl, hydroxyl, amino, cyano, nitro, halogenated C₁₋₄ alkyl, -NHC(O)R¹¹, benzyloxy, halogenated phenylmethoxy, aryl, and heteroaryl, R¹¹ is C₁₋₄ alkyl, or
for example, R¹⁰ is selected from the group consisting of fluorine, chlorine, bromine, iodine, methyl, ethyl, n-propyl, isopropyl, n-butyl, isobutyl, tert-butyl, n-pentyl, tert-pentyl, neopentyl, hexyl, trifluoromethyl, difluoromethyl, fluoromethyl, methoxy, ethoxy, n-propoxy, isopropoxy, n-butoxy, tert-butoxy, sec-butoxy, n-pentyloxy, n-hexyloxy, hydroxyl, amino, cyano, nitro, acetamido, formylamino, propionamido, benzyloxy, fluorophenylmethoxy, phenyl, pyridyl, pyrrolidinyl, cyclopentyl, cyclohexyl, morpholinyl, imidazolyl, and thiazolyl, or
for example, R¹⁰ is selected from the group consisting of fluorine, chlorine, bromine, methyl, ethyl, n-propyl, isopropyl, n-butyl, isobutyl, tert-butyl, trifluoromethyl, methoxy, ethoxy, n-propoxy, isopropoxy, n-butoxy, tert-butoxy, sec-butoxy, hydroxyl, amino, cyano, nitro, acetamido, formylamino, propionamido, benzyloxy, 4-fluorophenylmethoxy, phenyl, pyridin-2-yl, 5-bromopyridin-2-yl, cyclopentyl, and cyclohexyl.

7. The compound, a stereoisomer, a pharmaceutically acceptable salt, a pharmaceutically acceptable hydrate or solvate, or a pharmaceutically acceptable ester thereof according to claim 1, wherein R₁ is selected from the group consisting of cyclopentyl, thiazolyl and phenyl para- or meta-substituted by substituent R¹⁰, wherein R¹⁰ is selected from the group consisting of - NHC(O)R¹¹, C₁₋₆ alkoxy, benzyloxy and halogenated benzyloxy, R¹¹ is C₁₋₆ alkyl,
for example, R₁ is 4-acetamidophenyl, cyclopentyl, 4-(4-fluorobenzyloxy)phenyl, 3-benzyloxyphenyl, 4-propoxyphenyl, or thiazol-5-yl.

8. The compound, a stereoisomer, a pharmaceutically acceptable salt, a pharmaceutically acceptable hydrate or solvate, or a pharmaceutically acceptable ester thereof according to claim 1, wherein the compound is selected from the group consisting of:
N-{4-{7-[(2R,3R,4S,5R)-3,4-dihydroxy-5-(hydroxymethyl)tetrahydrofuran-2-yl]-7H-[1,2,4]triazolo[3,4-i]purin-3-yl}phenyl}acetamide;
(2R,3R,4S,5R)-2-{3-cyclopentyl-7H-[1,2,4]triazolo[3,4-i]purin-7-yl}-5-(hydroxymethyl)tetrahydrofuran-3,4-diol;
(2R,3R,4S,5R)-2-{3-[3,4-di(benzyloxy)phenyl]-7H-[1,2,4]triazolo[3,4-i]purin-7-yl}-5-(hydroxymethyl)tetrahydrofuran-3,4-diol;
(2R,3R,4S,5R)-2-{3-{4-[(4-fluorobenzyl)oxy]phenyl}-7H-[1,2,4]triazolo[3,4-i]purin-7-yl}-5-(hydroxymethyl)tetrahydrofuran-3,4-diol;
(2R,3R,4S,5R)-2-{3-[3-(benzyloxy)phenyl]-7H-[1,2,4]triazolo[3,4-i]purin-7-yl}-5-(hydroxymethyl)tetrahydrofuran-3,4-diol;
(2R,3R,4S,5R)-2-{3-[2,4-di(trifluoromethyl)phenyl]-7H-[1,2,4]triazolo[3,4-i]purin-7-yl}-5-(hydroxymethyl)tetrahydrofuran-3,4-diol;
(2R,3S,4R,5R)-2-{3-[4-(pyridin-2-yl)phenyl]-7H-[1,2,4]triazolo[3,4-i]purin-7-yl}-5-(hydroxymethyl)tetrahydrofuran-3,4-diol;
(2R,3R,4S,5R)-2-{3-{[1,1'-biphenyl]-4-yl}-7H-[1,2,4]triazolo[3,4-i]purin-7-yl}-5-(hydroxymethyl)tetrahydrofuran-3,4-diol;
(2R,3S,4R,5R)-2-{3-(4-propoxyphenyl)-7H-[1,2,4]triazolo[3,4-i]purin-7-yl}-5-(hydroxymethyl)tetrahydrofuran-3,4-diol;
(2R,3S,4R,5R)-2-{3-[4-(trifluoromethyl)phenyl]-7H-[1,2,4]triazolo[3,4-i]purin-7-yl}-5-(hydroxymethyl)tetrahydrofuran-3,4-diol;
(2R,3R,4S,5R)-2-{3-(5-bromopyridin-2-yl)-7H-[1,2,4]triazolo[3,4-i]purin-7-yl}-5-(hydroxymethyl)tetrahydrofuran-3,4-diol;
(2R,3S,4R,5R)-2-{3-(thiazol-5-yl)-7H-[1,2,4]triazolo[3,4-i]purin-7-yl}-5-(hydroxymethyl)tetrahydrofuran-3,4-diol; and
(2R,3R,4S,5R)-2-{3-cyclohexyl-7H-[1,2,4]triazolo[3,4-i]purin-7-yl }-5-(hydroxymethyl)tetrahydrofuran-3,4-diol.

9. A method for preparing the compound, a stereoisomer, a pharmaceutically acceptable salt, a pharmaceutically acceptable hydrate or solvate, or a pharmaceutically acceptable ester thereof according to any one of claims 1 to 8, comprising: 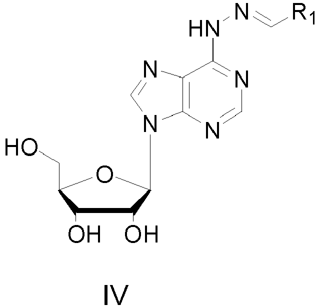 allowing a compound represented by Formula IV to undergo a cyclization reaction to obtain the compound represented by Formula (I),
preferably, the cyclization reaction is carried out in an acetic acid solution and in the presence of NBS,
preferably, the cyclization reaction is carried out at room temperature;
preferably, the compound represented by Formula IV is obtained by reacting a compound represented by Formula II with a substituted formaldehyde represented by Formula III, 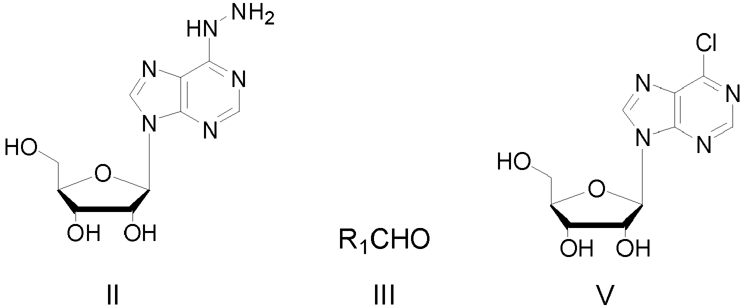
preferably, the compound represented by Formula II reacts with the substituted formaldehyde represented by Formula III in a methanol solution under microwave at 70°C to 90°C;
preferably, the compound represented by Formula II is obtained by the hydrazinolysis reaction of 6-chloroadenosine represented by Formula V with hydrazine hydrate,
preferably, the hydrazinolysis reaction is carried out at 60°C to 80°C, wherein the definition of R₁ is the same as that described in claims 1 to 6.

10. A pharmaceutical composition comprising at least one of the compound, a stereoisomer, a pharmaceutically acceptable salt, a pharmaceutically acceptable hydrate or solvate, or a pharmaceutically acceptable ester thereof according to any one of claims 1 to 8, and one or more pharmaceutically acceptable carriers or excipients.

11. Use of the compound, a stereoisomer, a pharmaceutically acceptable salt, a pharmaceutically acceptable hydrate or solvate, or a pharmaceutically acceptable ester thereof according to any one of claims 1 to 8, or the pharmaceutical composition according to claim 10 in the manufacture of a medicament as an A_{2A} adenosine receptor antagonist, or
in the manufacture of a medicament for preventing or treating a human pathological condition or symptom, wherein the human pathological condition or symptom is improved by antagonizing the A_{2A} adenosine receptor.

12. The use according to claim 11, wherein the pathological condition or symptom is selected from the group consisting of ischemia, supraventricular arrhythmia, atrial fibrillation, acute renal failure, myocardial reperfusion injury, disease caused by fluid retention, allergic reaction, scleroderma, arthritis, inflammatory bowel disease, diabetes, obesity, Parkinson's disease, Huntington's disease, dystonia, dyskinesia, congestive heart failure, hypertension, dialysis hypotension, dementia, anxiety disorder, glaucoma, and alcoholism.

13. A method for preventing and/or treating a human pathological condition or symptom, comprising administering to a patient in need thereof a therapeutically effective amount of the compound represented by Formula (I), a stereoisomer, a pharmaceutically acceptable salt, a pharmaceutically acceptable hydrate or solvate, or a pharmaceutically acceptable ester thereof according to any one of claims 1 to 8, or the pharmaceutical composition according to claim 10, wherein the human pathological condition or symptom is improved by antagonizing A_{2A} adenosine receptor.

14. The compound represented by the general Formula (I), a stereoisomer, a pharmaceutically acceptable salt, a pharmaceutically acceptable hydrate or solvate, or a pharmaceutically acceptable ester thereof according to any one of claims 1 to 8 for use in the prevention and/or treatment of a human pathological condition or symptom, wherein the human pathological condition or symptom is improved by antagonizing A_{2A} adenosine receptor, or
for use as an A_{2A} adenosine receptor antagonist.

15. The method according to claim 13 or the compound represented by general Formula (I), a stereoisomer, a pharmaceutically acceptable salt, a pharmaceutically acceptable hydrate or solvate, or a pharmaceutically acceptable ester thereof according to claim 14, wherein the human pathological condition or symptom is selected from the group consisting of ischemia, supraventricular arrhythmia, atrial fibrillation, acute renal failure, myocardial reperfusion injury, disease caused by fluid retention, allergic reaction, scleroderma, arthritis, inflammatory bowel disease, diabetes, obesity, Parkinson's disease, Huntington's disease, dystonia, dyskinesia, congestive heart failure, hypertension, dialysis hypotension, dementia, anxiety disorder, glaucoma, and alcoholism.
